# EUROPEAN PATENT SPECIFICATION

(11) **EP 1 747 755 B1**
(45) Date of publication and mention of the grant of the patent: **04.07.2018**
(21) Application number: 05739291.2
(22) Date of filing: 16.05.2005
(51) Int. Cl.: A61B 5/154, A61B 5/15

(54) **BACKFLOW PREVENTING STRUCTURE OF BLOOD SAMPLER, LURE NEEDLE, BLOOD SAMPLING NEEDLE AND BLOOD SAMPLING HOLDER**
RÜCKFLUSS-VERHINDERNDE STRUKTUR EINER BLUTPROBENENTNAHMEVORRICHTUNG, LUER-NADEL, BLUTENTNAHMENADEL UND BLUTPROBENHALTER
STRUCTURE ANTIREFLUX POUR DISPOSITIF DE PRÉLÈVEMENT SANGUIN, AIGUILLE LUER LOCK, AIGUILLE POUR PRÉLÈVEMENT SANGUIN ET PORTE-PRÉLÈVEMENTS

(30) Priority: 17.05.2004 JP 2004146985
(43) Date of publication of application: 31.01.2007
(73) Proprietor: SEKISUI CHEMICAL CO., LTD., Osaka-shi, Osaka 530-8565 (JP)
(72) Inventor: Nakaizumi, Masahiro, Shunan-shi, Yamaguchi 7460006 (JP); Okamoto, Ryusuke, Shunan-shi, Yamaguchi 7460006 (JP)
(74) Representative: Ter Meer Steinmeister & Partner
(86) International application number: PCT/JP2005/008895
(87) International publication number: WO 2005/110224

(56) References cited:
- WO-A1-01/64265
- JP-A- 53 097 289
- JP-A- 2002 510 984
- US-A- 2 840 112
- US-A- 3 675 658
- US-A- 3 874 367
- US-A- 4 244 379
- US-A- 4 307 731
- US-A- 4 540 027
- US-A- 5 245 991
- US-A- 5 855 547

## Description

### TECHNICAL FIELD

The present invention relates to a backflow preventing structure of a blood sampler used for sampling blood, and more specifically, to a backflow preventing structure, and a luer adapter, a blood sampling needle and a blood sampling holder having a backflow preventing structure.

### BACKGROUND ART

In current blood sampling methods such as vacuum blood sampling, a variety of blood sampling needles and blood sampling holders are used. In sampling of blood, first a tourniquet is attached to an arm of a subject whose blood is to be sampled. Then a needlepoint of a blood sampling hollow needle is inserted into a blood vessel. The blood sampling hollow needle is connected with a communicating hollow needle for piercing a plug member of a vacuum blood sampling tube. Various structures such as a hub having an internal channel have been proposed as the structure that connects the blood sampling hollow needle and the communicating hollow needle.

In conducting vacuum blood sampling, after inserting the needlepoint of the blood sampling hollow needle into the blood vessel as described above, the plug member of the vacuum blood sampling tube is pierced by the needlepoint of the communicating hollow needle. This makes the blood sampling hollow needle and the interior of the vacuum blood sampling tube in communication with each other. Since the interior of the vacuum blood sampling tube is depressurized, blood is introduced from the blood vessel to the vacuum blood sampling tube.

Blood sampling completes when the internal pressure of the vacuum blood sampling tube and the pressure of the blood vessel come are almost nearly equivalent with each other. After completion of blood sampling, the communicating hollow needle is withdrawn from the vacuum blood sampling tube, and the tourniquet is removed. However, if the tourniquet is erroneously removed in first, the blood in the blood sampling tube may possibly backflow toward the blood vessel due to a sudden drop in internal pressure of the blood vessel. When the blood sampling tube is placed at a higher position than the blood vessel, the potential energy may cause backflow. On the other hand, a vacuum blood sampling tube often contains an anticoagulant, a medical agent and the like. Therefore, if backflow occurs, such an anticoagulant and a medical agent move toward the blood vessel to exert an adverse effect. In view of the above, various structures for preventing backflow have been proposed heretofore.

For example, Patent document 1 recited blow discloses a blood sampling needle 101 shown in Fig. 17. The blood sampling needle 101 includes a first hollow needle 102 to be inserted into a blood vessel for collecting blood, and a second hollow needle 103 for piercing a plug member of a vacuum blood sampling tube and communicating with the interior of the vacuum blood sampling tube. The first and the second hollow needles 102, 103 are secured to a hub 104 on their proximal sides of the first and second hollow needles 102, 103. In the hub 104, an internal channel 104a is formed. The internal channel 104a communicates with the first and the second hollow needles 102, 103. In order to prevent backflow, a check valve 105 made of a flexible resin or the like is formed in the internal channel 104a. In the check valve 105, a plurality of valve members are arranged such that their intervals decrease as they come closer to the second hollow needle 103 provided for communication.

Therefore, when blood flows within the internal channel 104a from the first hollow needle 102 toward the second hollow needle 103, a gap of the check valve 105 opens to allow the blood pass through. To the contrary, when blood is about to flow in the counter direction, the gap in tip end is closed by the pressure of blood, whereby backflow of the blood is prevented.

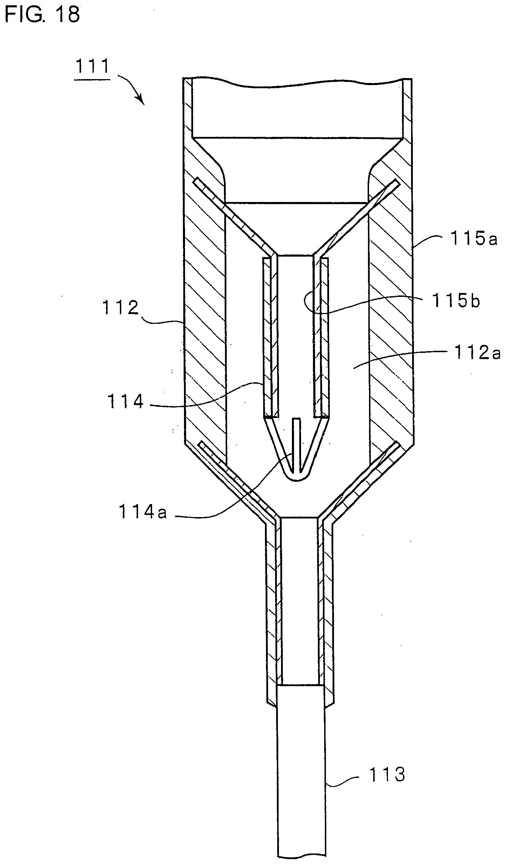

In the meanwhile, Patent document 2 recited below discloses a blood sampling needle 111 equipped with a backflow preventing structure shown in Fig. 18. The blood sampling needle 111 has a hub 112. The hub 112 has an internal channel 112a whose one end is in communication with a blood sampling needle which is to be inserted into a blood vessel. The other end of the internal channel 112a is connected with a hollow needle 113 which is brought into communication with a vacuum blood sampling tube. In the internal channel 112a, a backflow preventing member 114 is provided for allowing blood to flow toward the hollow needle 113 but not in the counter direction. The backflow preventing member 114 is made from a tubular elastic body, and a cut 114a is provided in the tip end of the backflow preventing member 114. The cut 114a has a cross shape when viewed from lower end side of Fig. 18. In blood sampling, the cut 114a opens due to blood flow, and blood flows toward the hollow needle 113. However, when blood is about to backflow, the cross-shaped cut 114a is narrowed by force of blood flow in the elastic backflow preventing member 114, so that backflow is prevented.

Fig 4 of Patent document 3 recited blow discloses a backflow preventing structure in a blood sampling part utilizing a valve made of an elastic material. Here, the valve made of an elastic material is formed with a slit extending in the flow path direction of the blood sampling part, namely in the axial direction of the tubular body. This ensures that the slit is closed by backflow if such backflow occurs.
Patent document 1: Japanese Patent Application Laid-open Publication No. 03-129111
Patent document 2: Japanese Patent Application Laid-open publication No. 2003-260132
Patent document 3: Japanese Patent Application Laid-open publication No. 50-12892

In the blood sampling needle 101 disclosed in the Patent document 1, backflow was prevented by providing the backflow preventing valve 105 in the internal channel 104a. However, when the backflow preventing valve 105 has a conical shape, an outlet for blood is very narrow, so that hemolysis is likely to occur during passage of blood although backflow is prevented. Additionally, when such check valve 105 is made by molding, another problem arises that miniaturization is difficult to be achieved.

In the blood sampling needle 111 disclosed in Patent document 2, the backflow preventing member 114 having the cross cut 114a formed by cutting the tip end in a cross shape is used. In this case, it is necessary to accurately cut an elastic member from bottom end side into a crossing cut. This increases the processing steps, and hence such measure is not appropriate for production of blood sampling needles that are consumed in the order of 0.5 to 10 billions every year. Additionally, since a blood sampling needle is a disposable component, the cost of blood sampling needle necessarily rises when such a needle that has a crossing cut as is the case of the backflow preventing member 114 is used. Additionally, as shown in Fig. 18, the backflow preventing member 114 formed from a tubular body having a cut 114a is inserted on and secured to a cylindrical part 115b that is connected with a securing member 115a. Therefore, aside from the backflow preventing member 114, the securing member 115a requires other members of complicated shape such as cylindrical part 115b, which necessitate a cost increase.

In the structure disclosed in Patent document 3 in which a valve of elastic material having a slit is used, when the pressure of backflow is high, the valve parts on each end of the slit is pushed inside by backflow to open the slit, which leads failure in secure prevention of backflow.

US 3,874,367 A discloses a valve having a tubular body or sleeve with one end closed. The valve is made of a self-sealing elastomeric material and contains one or more slits where a hollow portion of it tubular body is located. The slits extend in a longitudinal direction of the tubular body. The valve is of a lesser inner diameter than the outer diameter of a cannula so that when the valve is positioned on cannula it is expanded at the location of contact so as to provide sealing inter-engagement between the cannula and valve sleeve. When blood flows in one direction the slits open and blood can pass the valve, whereas in the opposite direction the slit closes and hence the valve blocks the blood flow.

US 4,540,027 discloses a backflow preventing structure of an infusion or transfusion apparatus for preventing backflow. The structure comprises a casing, the lower part of which forms a tubular body having an internal channel extending from a first end part toward a second end part through which a liquid flows, and a backflow preventing member or valve contained in the internal channel of the tubular body. The valve is made of a material having rubber elasticity. The valve being a bottomed tube closed at the second end part side and having subsequent to the flat bottom and a cylindrical main body part and a thickened part upwardly subsequent to the main body part with an opening that opens toward the first end part. The thickened part has an extended outer diameter when compared to the main body part. A cut is made in the cylindrical main body part of the backflow preventing member at a part closer to the second end part side then the thickened part, wherein the cut extends in a direction that intersects with a direction connecting the first end part and the second end part, to form a hinge part. The hinge part has a thickness increasing in a circumferential direction from zero to a maximum value equal to the thickness of the wall of the main body part and decreases thereafter to zero.

US 4,244,379 shows a check valve for a blood drawing apparatus. The check valve includes a main body member having a longitudinally extending cavity as internal channel. A backflow preventing valve member is inserted into the internal channel. The valve member is formed as a bottomed tube semispherically closed at one end and having subsequent to the semispherical bottom end a cylindrical part and a skirt portion. A longitudinal slit is provided in the cylindrical part. To fix the valve member within the internal channel of the body member, a plug carrying a needle is inserted into the internal channel so as to clamp the skirt portion between an end portion of the plug and a corresponding shoulder provided in the tubular body part like a conventional flange

US 5,855,547 discloses a vacuum pump having a bulb, the wall of which is composed of elastic material and having a first protuberance as an air intake passageway and a second protuberance projecting outwardly of the bulb having an air outlet passageway. The protuberances are integrally formed with the elastic material of the bulb and are formed as tubes having a semispherically closed end. A cut is provided so that the protuberances can serve as check valves allowing airflow in the vacuum bulb pump to be operated. Due to the cut a hinge part is formed which has the form of a segment of a circle.

US 3,675,658 is concerned with a catheter assembly having a valved fluid reservoir. The valve for closing the outlet of the reservoir consists of a bottomed tube semispherically closed at its inlet formed by a cut and having subsequent to the semispherical bottom or inlet a cylindrical body part with an opening that opens toward the outlet side of the valve.

WO 01/64265 A1 discloses a tube dependent anti-free flow valve. The valve device comprises a hollow flexible body having tapered portion. The tapered portion includes opposing beveled surfaces. The body further includes a proximal end that forms an outlet in communication with a passage such that fluid, which enters the tapered portion, may pass along passage and through outlet. To permit entering of a fluid through the tapered portion a slit is formed by cutting the tapered portion in a direction along a direction connecting the tapered portion and the proximal end of the body.

### DISCLOSURE OF THE INVENTION

It is an object of the present invention to provide a backflow preventing structure suitable for preventing backflow of blood in a blood sampler which securely prevents backflow during blood sampling, while eliminating a risk of hemolysis and enabling provision at low cost without causing increase in process steps.

It is another object of the present invention to provide as a blood sampler equipped with the above backflow preventing structure of a blood sampler, a luer adapter, a blood sampling needle and a blood sampling holder.

These objects are achieved by the subject matter of the independent claims.

In one specific aspect of the backflow preventing structure of a blood sampler according to the present invention, the cut is provided in at least two positions in the backflow preventing member.

In other specific aspect of the backflow preventing structure of a blood sampler according to the present invention, the cut formed in the backflow preventing member extends in a direction inclined at an angle ranging from 15 to 165 degrees with respect to the direction connecting the first end part and the second end part, i.e., with respect to the extending direction of the internal channel.

In a further specific aspect of the backflow preventing structure of a blood sampler according to the present invention, the cut formed in the backflow preventing member extends in a direction perpendicular to the direction connecting the first end part and the second end part.

In other specific aspect of the backflow preventing structure of a blood sampler according to the present invention, a part that serves as a hinge for opening/closing achieved by the cut has a relatively smaller thickness than the remaining part.

A luer adapter according to the present invention includes a hollow needle used for piercing a plug member of a blood sampling tube in blood sampling, and a hub secured to one end of the hollow needle, wherein the hub has the backflow preventing structure of a blood sampler configured according to the present invention, and the hollow needle is in communication with the second end part side of the internal channel of the backflow preventing structure of a blood sampler.

A blood sampling needle according to the present invention includes a hollow needle for collecting blood; and a hub secured to one end of the hollow needle, wherein the hub has the backflow preventing structure of a blood sampler configured according to the present invention, and the hollow needle is in communication with the first end part side of the internal channel of the backflow preventing structure of a blood sampler.

A blood sampling holder of the present invention includes a tubular holder main body formed with an opening for inserting a blood sampling tube at its one end, and closed at the other end; a hub provided on the other end side of the holder main body; and a hollow needle secured at its one end to the hub, for piercing a plug member of the blood sampling tube, wherein the hub has the backflow preventing structure of a blood sampler configured according to the present invention, and the hollow needle is in communication with the second end part side of the internal channel of the backflow preventing structure of a blood sampler.

In one specific aspect of the blood sampling holder according to the present invention, a blood sampling hollow needle having one end secured to the hub and the other end extended outside the holder is further included, wherein the blood sampling hollow needle is in communication with the first end part side of the internal channel of the backflow preventing structure of a blood sampler.

In the backflow preventing structure of a blood sampler of the present invention, the backflow preventing member is contained in the internal channel of the tubular body having the internal channel extending from the first end part toward the second end part through which blood flows, and the backflow preventing member is made of a material having rubber elasticity, and has a shape of a bottomed tube closed on the second end part side and having an opening that opens toward the first end part; and the outer circumferential wall of the backflow preventing member is secured liquid-tightly to the inner wall of the internal channel; and the cut is made in the backflow preventing member at a part closer to the second end part side than the secured part.

Accordingly, when blood flows from the first end part toward second end part in the internal channel, the cut provided in the backflow preventing member made of a material having rubber elasticity is opened by being pushed by the blood, to allow passage of the blood. In this case, since the backflow preventing member is made of a flexible material having rubber elasticity, the cut is readily opened, so that hemolysis is unlikely to occur.

On the other hand, when the blood is about to backflow from the second end part toward the first end part, the cut is closed by force of blood flow. Also in this case, since the backflow preventing member is made of a flexible material having rubber elasticity, the cut is securely closed by force of blood flow, so that backflow is securely prevented.

Since all that is necessary is to form a cut in the backflow preventing member which is a bottomed tube made of a material having rubber elasticity, and to secure it in the internal channel, it is possible to provide an inexpensive backflow preventing structure without causing a significant increase in number of process steps and components.

In the backflow preventing member, when cuts are provided in at least two positions, the backflow preventing member is secured aslant in the internal channel of the tubular body, and at least one of the cuts comes into close contact with the inner wall of the tubular body. Accordingly, even if one of the cuts fails to open, the remaining at least one cut will securely open to enable blood sampling.

When the cut is formed in the backflow preventing member in the direction that intersects with the direction connecting the first and the second end parts, the cut part is closed more securely by force of blood flow in the case of backflow. Additionally, only by forming a cut in the direction that intersects with the direction connecting the first and the second end parts, it is possible to readily form a backflow preventing cut.

Furthermore, when the direction in which the cut extends is inclined so as to form an angle ranging from 15 to 165 degrees with respect to the direction connecting the first and the second end parts, namely, the direction of blood flow in the internal channel, an opening area is increases when the corresponding portion is open. Consequently, the blood will flow smoothly via the opening, and the blood flow resistance will be reduced. Therefore, even when the backflow preventing member is used, it is possible to prevent the blood sampling time from being undesirably extended.

In the situation that the cut extends in the direction that is perpendicular to the direction connecting the first and the second end parts, when blood is about to backflow from the second end part toward the first end part, the cut part is more securely closed by backflow force. Therefore, it is possible to prevent backflow from occurring more effectively.

In the above backflow preventing member, when the part functioning as a hinge during opening/closing achieved by the cut has smaller thickness than the remaining part of the backflow preventing member, the cut will securely open only with application of a relatively small pressure, so that rapid blood sampling is realized. Furthermore, by making only the hinge part thinner, the area of the part opposing via the cut is large enough. Therefore, even if the cut part is closed while the opposite parts slightly misaligned, the cut can be securely closed.

According to the luer adapter of the present invention, in the luer adapter having a hollow needle used for piercing a plug member of a blood sampling tube and a hub secured to one end of the hollow needle, the hub has the backflow preventing structure configured in accordance with the present invention, and the hollow needle is communicated with the second end part side of the internal channel of the backflow preventing structure. Therefore, it is possible to securely prevent backflow in the luer adapter by the backflow preventing structure of the present invention, and to provide a luer adapter equipped with a backflow preventing structure at low cost without significantly increasing the number of components.

According to the blood sampling needle of the present invention, in a structure having a hollow needle with one end to be inserted into a blood vessel or container containing blood for sampling blood, and a hub to which one end of the hollow needle is secured, the hub has the backflow preventing structure configured according to the present invention, and the hollow needle is communicated with the first end part side of the internal channel.

Therefore, when blood flows from the second end part side toward the first end part side, namely flows back, the cut provided in the backflow preventing structure is securely closed by force of the blood flow, so that backflow is securely prevented. In addition, since the backflow preventing structure of the present invention can be obtained without causing significant increase in number of process steps and components, the blood sampling needle having the above backflow preventing structure can be provided at low cost.

According to the blood sampling holder of the present invention, in the blood sampling holder including a holder main body formed with an opening for insertion of a blood sampling tube at one end side and closed at the other end side; a hub provided on the other end side; and a hollow needle for piercing a plug member of the blood sampling tube, the hub has the backflow preventing structure configured according to the present invention, and the hollow needle is communicated with the second end part side of the internal channel of the backflow preventing structure of the blood sampling part. Therefore, it is possible to securely prevent backflow by means of the backflow preventing structure, and to prevent backflow without causing a significant increase in number of components. Therefore, it is possible to provide an inexpensive and safe blood sampling holder.

The shape of the cut is not limited to those described above. Examples are shown in attached drawings. For these shapes, the cut may also be formed at only one position or at plural positions. Further, the cut may be rotated axially at any angle. The cut amount is appropriately adjusted to optimum depending on the shape and number of cut.

When the blood sampling holder of the present invention further includes a blood sampling hollow needle having one end secured to the hub and the other end extending outside the holder, and the blood sampling hollow needle is communicated with the first end part side of the internal channel of the backflow preventing structure, it is possible to provide a blood sampling holder having excellent safety in which the hollow needle is integrated with the hollow needle provided for connection to the blood sampling tube according to the present invention.

### BRIEF EXPLANATION OF DRAWINGS

[Figure 1] Figs. 1(a) and 1(b) are views for explaining a blood sampler according to a first embodiment of the present invention, wherein Fig. 1(a) is a front view of a blood sampling holder and Fig. 1(b) is a front section view for explaining the process of securing a blood sampling needle to a blood sampling holder.
[Figure 2] Figs. 2(a) and 2(b) are respectively, a perspective view of a backflow preventing member used in the first embodiment, and a perspective view in a state that a cut is opened by blood.
[Figure 3] Fig. 3 is a schematic plan section view for explaining a preferred size range of cut formed in the backflow preventing member according to the present invention.
[Figure 4] Fig. 4 is a front section view for illustrating the step of sampling blood with the use of the blood sampler according to the first embodiment of the present invention.
[Figure 5] Figs. 5(a) and 5(b) are partially cutaway front section views respectively showing the state in which blood is collected while the cut of the backflow preventing member according to the first embodiment is open and the state that the cut is blocked by backflow.
[Figure 6] Fig. 6 is a longitudinal section view showing a blood sampler according to a second embodiment of the present invention.
[Figure 7] Fig. 7 is a front section view showing a blood sampling needle which is a third embodiment of the present invention.
[Figure 8] Fig. 8 is a front section view showing the state in which the blood sampling needle of the third embodiment is secured to a blood sampling holder.
[Figure 9] Figs. 9(a) and 9(b) are schematic front section views for explaining a luer adapter according to a fourth embodiment of the present invention.
[Figure 10] Figs. 10(a) and 10(b) are schematic front section views for explaining a luer adapter according to a fifth embodiment of the present invention.
[Figure 11] Fig. 11 is a schematic front section view for explaining a luer adapter according to a sixth embodiment of the present invention.
[Figure 12] Figs. 12(a) and 12(b) are partially cutaway views for explaining an alternate example of the luer adapter shown in Fig. 9(a).
[Figure 13] Figs. 13(a) and 13V are partially cutaway views for explaining an alternate example of the luer adapter shown in Fig. 9(a).
[Figure 14] Fig. 14 is a partially cutaway enlarged front section view for explaining a preferred dimensional relationship of the luer adapter according to a third embodiment.
[Figure 15] Fig. 15 is a schematic front section view for explaining an evaluation method of backflow amount using a luer adapter in Examples 1 to 4.
[Figure 16] Figs. 16(a) to 16(c) are schematic section views showing alternate examples of shape of cut in the backflow preventing member according to the present invention.

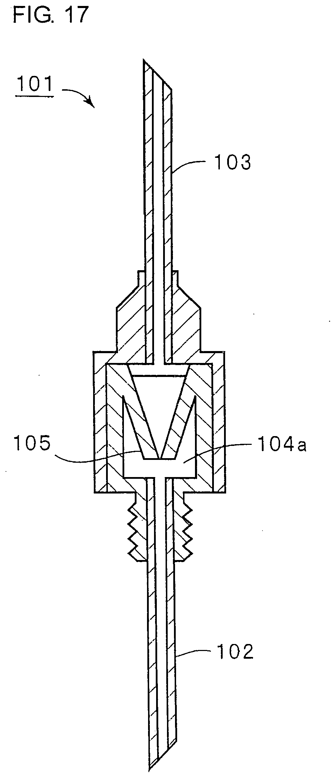

[Figure 17] Fig. 17 is a front section view showing one exemplary conventional blood sampling needle.
[Figure 18] Fig. 18 is a front section view showing another exemplary conventional blood sampling needle.

### EXPLANATION OF REFERANCE NUMERALS

- 1: blood sampling holder
- 2: holder main body
- 2a: opening
- 2b: end
- 3: hub
- 3a: internal channel
- 3b: first end part
- 3c: second end part
- 4: hollow needle
- 4a: needlepoint
- 5: rubber sheath
- 6: bottomed tubular backflow preventing member
- 6a: thickened part
- 6b: main body part
- 6c: cut
- 6d: flange part
- 6f: cut
- 6g: flange part
- 11: hollow needle
- 11a: needlepoint
- 12: needle fixing part
- 31: blood sampling holder
- 31a: opening
- 31b: end surface
- 31c: female screw
- 33: hub
- 33a: internal channel
- 33b: first end part
- 33c: second end part
- 34: hollow needle
- 41: blood sampling needle
- 43: hub
- 43a: internal channel
- 43b: first end part
- 43c: second end part
- 44: hollow needle
- 44a: needlepoint
- 45: hollow needle
- 45a: needlepoint
- 51: luer adapter
- 52: rubber sheath
- 53: luer adapter
- 54: luer adapter
- 61: luer adapter
- 62: luer adapter

### BEST MODE FOR CARRYING OUT THE INVENTION

The present invention will be more apparent by referring the following explanation of concrete embodiments of the present invention taken together with the attached drawings.

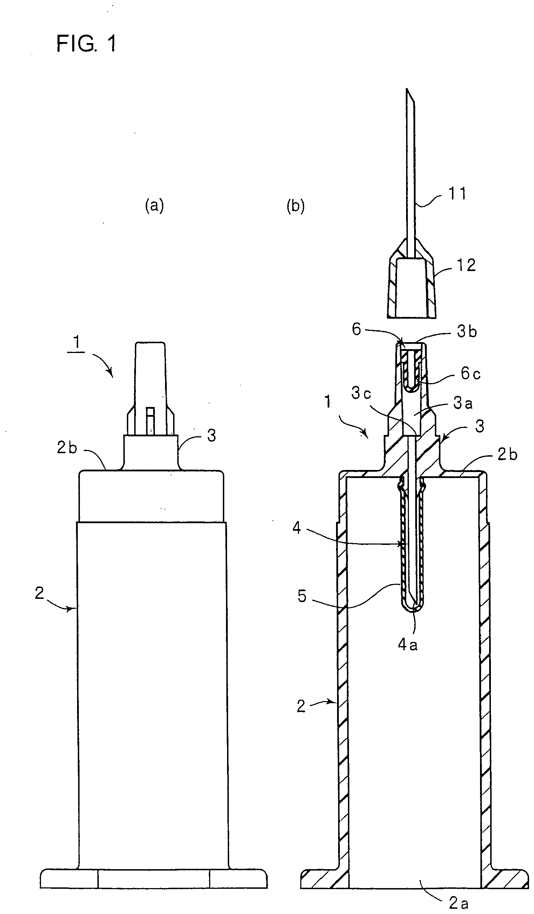

Figs. 1(a) and 1(b) are a front view and a front section view for explaining a blood sampling holder equipped with a backflow preventing structure of a blood sampler according to one embodiment of the present invention.

A blood sampling holder 1 shown in Fig. 1(a) has a holder main body 2 of a circular tubular shape. As shown in Fig. 1(b), the holder main body 2 has an opening 2a on its one end. As will be described later, the opening 2a is dimensioned such that a vacuum blood sampling tube is inserted through the opening 2a. The other end on the opposite side of the opening 2a is closed, and on the side of the other end, a hub 3 is formed in integration with the cylindrical holder main body 2. The hub 3 which is a cylindrical portion having a smaller diameter than the holder main body 2 is provided so as to protrude outside from an end surface 2b of the holder main body 2. In the hub 3, an internal channel 3a is formed. In other words, according to the present embodiment, the hub 3 corresponds to a tubular body constituting the backflow preventing structure, and the internal channel 3a is formed in this tubular body. The internal channel 3a extends from a first end part 3b toward a second end part 3c situated on the opposite side. The first end surface 3b of the internal channel 3a opens at a distal end of the hub 3.

A hollow needle 4 is secured to the hub 3 in such a manner that it is in communication with the second end part 3c of the internal channel 3a. The hollow needle 4 is arranged such that a needlepoint 4a at its distal end extends inside the holder main body 2. On the periphery of the hollow needle 4, a rubber sheath 5 is attached.

The hollow needle 4 is provided for piercing a plug member of a vacuum blood sampling tube. To be more specific, in conducting a vacuum blood sampling, by piercingly inserting the hollow needle 4 through a plug member, communication between the interior of the blood sampling tube and the above internal channel 3a, and therefore communication between a later-described blood sampling needle and a blood vessel are established.

The rubber sheath 5 is made of a rubber material having flexibility, and provided for preventing blood from leaking outside at the needlepoint 4a at the distal end of the hollow needle 4. When the plug member is pierced with the needlepoint 4a, the rubber sheath 5 is also pierced with the needlepoint 4a. As a result, the rubber sheath 5 comes into close contact with the outer circumferential face of the hollow needle 4, so that it is possible to prevent blood from leaking outside the blood sampling tube. It is to be noted that the rubber sheath 5 is not necessarily provided.

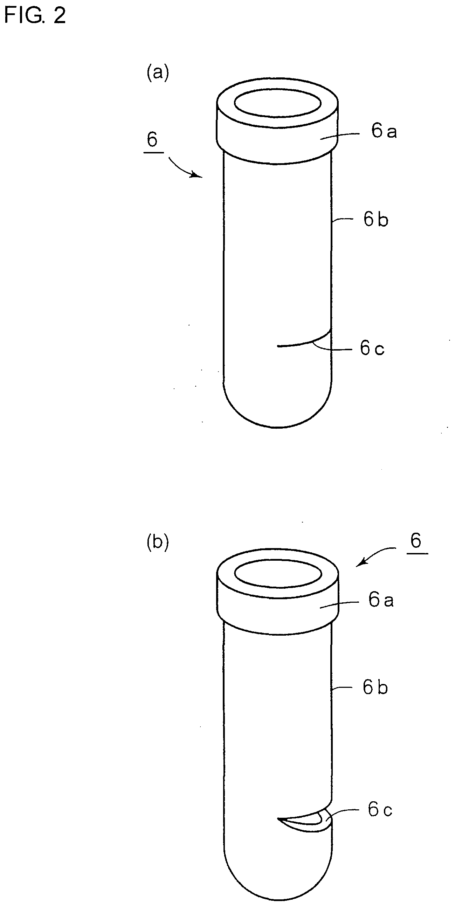

The internal channel 3a contains a backflow preventing member 6. As shown in an enlarged scale in Fig. 2(a), the backflow preventing member 6 is formed of a bottomed tubular member. The backflow preventing member 6 is formed of a material having rubber elasticity and hence has flexibility.

In the present embodiment, the backflow preventing member 6 has a substantially circular tubular shape, and has near its open end a thickened part 6a having an extended outer diameter. The diameter of a main body part 6b that is downwardly subsequent to the thickened part 6a is designed to be relatively small, and the main body part 6b is formed with a cut 6c. The cut 6c is provided so as to penetrate through a lateral surface of the backflow preventing member 6. The cut 6c may be readily formed by simply cutting the backflow preventing member 6 formed of a material having rubber elasticity with a cutter or the like, for example.

In the present embodiment, the cut 6c is formed so as to extend in the direction perpendicular to the longitudinal direction of the backflow preventing member 6.

Referring again Fig. 1, the backflow preventing member 6 is contained in such a manner that the opening end, namely, the side of the thickened part 6a is located on the first end part 3b side of the internal channel 3a. Herein, the outer diameter of the thickened part 6a is slightly larger than the inner diameter on the end side of the internal channel 3a so that the backflow preventing member 6 may be pressed into the internal channel 3a.

The backflow preventing member 6 is inserted and pressed into the internal channel 3a through a closed portion on the opposite side of the thickened part 6a. As a result, the outer circumferential surface of the thickened part 6a comes into close contact with the inner circumferential surface of the internal channel 3a. Accordingly, the backflow preventing member 6 can be secured within the internal channel 3a only by insertion into the internal channel 3a.

The cut 6c is situated at a position closer to the second end part 3c than the part where the backflow preventing member 6 is secured to the internal channel 3a, or than the thickened part 6a in the present embodiment. Since the cut 6c is formed in a direction perpendicular to the longitudinal direction of the backflow preventing member 6, it also extends in a direction perpendicular to the direction connecting the first end part 3b and the second end part 3c of the internal channel 3a.

Examples of the rubber elastic material forming the backflow preventing member 6 include synthetic rubbers such as silicone rubber, butyl rubber, and thermosetting elastomer, as well as natural rubber.

Through explanation of a blood sampling method using the blood sampling holder 1 of the present embodiment, an operation and an effect of the backflow preventing structure equipped with the above backflow preventing member 6 will be explained.

Prior to blood sampling, a hollow needle 11 is attached to the hub 3 of the blood sampling holder 1 as shown in Fig. 1(b). The hollow needle 11 has a tubular securing part 12 on its proximal end side. The securing part 12 is inserted on the hub 3 and secured to the hub 3 in a liquid tight manner. As a result, the interior of the hollow needle 11 is brought into communication with the internal channel 3a on the first end part 3b side.

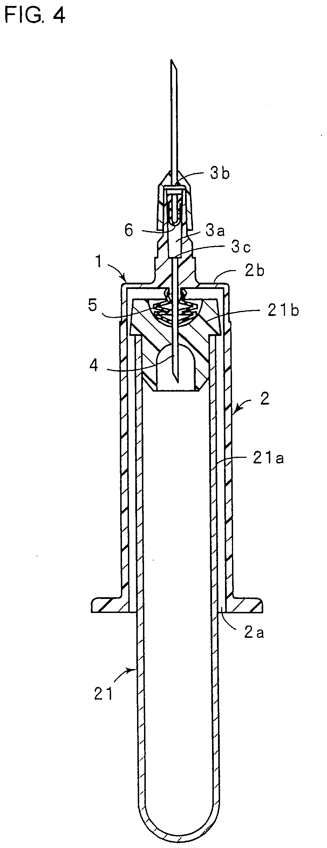

For blood sampling, a tourniquet is wore on an arm of a subject whose blood is to be sampled. After securing the hollow needle 11 on the hub 3 of the blood sampling holder 1, the needlepoint 11a of the hollow needle 11 is inserted into a blood vessel. Then as shown in Fig. 4, the blood sampling tube 21 is inserted through the opening 2a of the holder main body 2 of the blood sampling holder 1. The blood sampling tube 21 is a normal vacuum blood sampling tube, and has a blood sampling tube main body 21a of a bottomed circular tubular shape, and a plug member 21b having rubber elasticity. The plug member 21b is pierced with the hollow needle 4. As a result, as shown in Fig. 4, the internal channel 3a is brought into communication with the interior of the blood sampling tube 21. The interior of the blood sampling tube 21 is in a decompressed condition. Therefore, blood will flow toward the internal channel 3a of the hollow needle 11 due to a pressure difference between the inner pressure of the blood sampling tube 21 and the inner pressure of the blood.

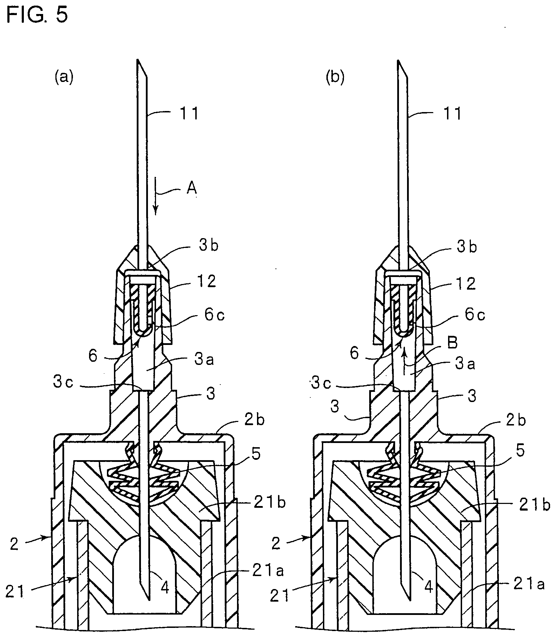

The backflow preventing member 6 has a bottomed circular tubular shape that opens toward the first end part. The cut 6c is opened by the pressure of blood that is guided thereto as shown in Fig. 2(b). In other words, as shown in Fig. 5(a), when blood flows toward the second end part 3c from the first end part 3b side in the direction of the arrow A, the cut 6c will open. Consequently, the blood flows down from the cut 6c of the backflow preventing member 6, passes through the hollow needle 4, and then guided to the interior of the blood sampling tube 21.

When the inner pressure of the blood sampling tube 21 comes into equilibrium with the inner pressure of the blood vessel, the blood sampling completes. Then, blood sampling can be finished by drawing the blood sampling tube 21 out of the blood sampling holder 1. In such a situation, if the tourniquet is erroneously removed in first, and the blood collected in the blood sampling tube 21 is about to flow back due to a sudden pressure drop in the blood vessel, the backflow is securely prevented by the backflow preventing member 6.

That is, as shown in Fig. 5(b), when the blood flows in the direction denoted by the arrow B due to occurrence of backflow, a lower end part, e.g., a part on the first end part side of the backflow preventing member 6 will be pushed toward the second end part side. As a result, the cut 6c is closed, so that blood is securely prevented from flowing farther from the backflow preventing member 6 toward the first end part 3b side.

Therefore, by using the blood sampling holder 1 of the present invention, it is possible to securely prevent blood from flowing back because of provision of the backflow preventing structure within the hub. According to this backflow preventing structure, all that is needed is to arrange the backflow preventing member 6 made of a material having rubber elasticity within the internal channel 3a, and the backflow preventing member 6 can be formed only by forming the cut 6a in the rectangular circular tubular body. Therefore, increases in processing steps and in number of components are difficult to be caused.

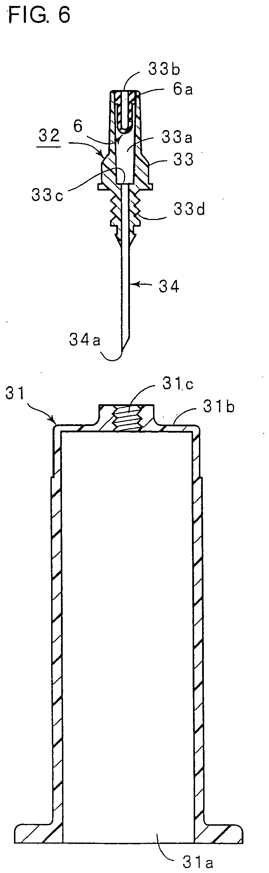

In the above embodiment, the backflow preventing structure is provided in the hub 3 that is formed in integration with the main body 2 of the blood sampling holder 1. However, the backflow preventing structure of the present invention may be applied to various types of blood samplers. This will be explained with reference to Figs. 6 to 8.

In a second embodiment shown in Fig. 6, a tubular blood sampling holder 31 and a luer adapter 32 are combined to achieve a structure corresponding to the blood sampling holder 1 of the first embodiment. In other words, the blood sampling holder 31 has a shape similar to that of the holder main body 2 of the first embodiment. On an end surface 31b on the opposite side of an opening 31a, a needle attachment part for attachment of a blood sampling needle 32 is provided. The needle attachment part has a through-hole having an outer circumferential surface on which a female screw 31c is formed. On the other hand, the luer adapter 32 has a hub 33, and a hollow needle 34 having one end secured to the hub 33. The hollow needle 34 is secured at its end opposite to the needlepoint 34a, to the hub 33. The hub 33 has an internal channel 33a. The internal channel 33a is formed along a direction connecting a first end part 33b and a second end part 33c. A proximal end of the hollow needle 34 is in communication with the second end part 33c. In a lower part of the hub 33, a male screw 33d is formed on the outer circumferential surface. The male screw 33d is designed to be screwed into the female screw 31c provided in the needle attachment part.

As is the case of the internal channel 3a in the first embodiment, the backflow preventing member 6 is pressed into the internal channel 33a, and the thickened part 6a of the backflow preventing member 6 is in close contact with an inner circumferential surface of the internal channel 33a in a liquid tight manner.

In the present embodiment, by inserting the luer adapter 32 into the blood sampling holder 31 from the hollow needle 34 side, and screwing the male screw 33d into the female screw 31c, the luer adapter 32 is secured to the blood sampling holder 31. As a result, a structure similar to that of the blood sampling holder 1 of the embodiment shown in Fig. 1 is obtained.

Therefore, by attaching the hollow needle 11 shown in Fig. 1A, it is possible to use them in a similar manner as described in the first embodiment. In other words, the blood sampler shown in Fig. 6 has a structure corresponding to that obtained by dividing the blood sampling holder 1 shown in Fig. 1 into the luer adapter 32 and the blood sampling holder 31.

In this manner, the backflow preventing structure of a blood sampler may be provided in the luer adapter 32 having the hollow needle 34 that pierces a plug member of a blood sampling tube.

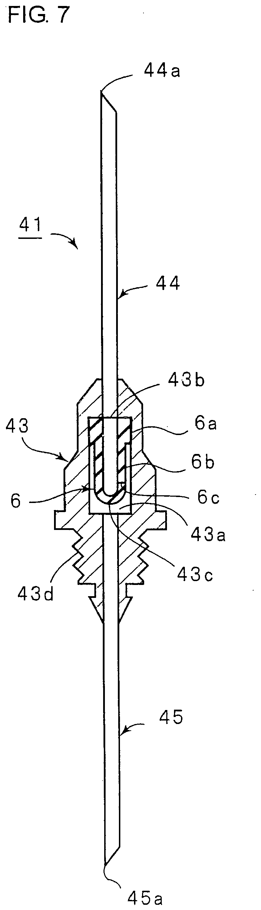

Fig. 7 is a front section view for explaining a third embodiment of the present invention. In the third embodiment, there is provided a blood sampling needle 41. The blood sampling needle 41 has such a structure that is obtained by integrating the luer adapter 32 shown in Fig. 6 and the hollow needle 11 shown in Fig. 1(b).

That is, the blood sampling needle 41 has a hub 43. The hub 43 has an internal channel 43a. The internal channel 43a is formed in the direction connecting a first end part 43b and a second end part 43c. The internal channel 43a contains a backflow preventing member 6. The backflow preventing member 6 is configured similarly to the backflow preventing member 6 shown in Fig. 1. Therefore, in the present embodiment, the backflow preventing structure of the present invention is arranged in the hub 43.

To one end of the hub 43, a proximal end of a first hollow needle 44 is secured. A needlepoint 44a of the first hollow needle 44 is a portion that is to be inserted into a blood vessel, and the proximal end is in communication with the internal channel 43a on the first end part 43b side. On the other hand, to the other end of the hub 43, a proximal end of a hollow needle 45 is secured, and the proximal end is in communication with the second end part 43c of the hollow needle 43. The hollow needle 45 is used in such a manner that it is pierced toward a plug member of a blood sampling tube from the side of a needlepoint 45a.

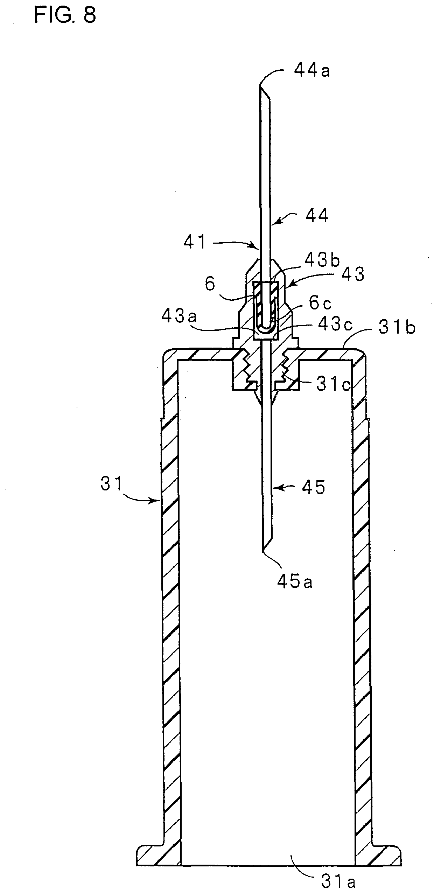

Therefore, it can be seen that the blood sampling needle 41 has a structure that is obtained by securing the blood sampling needle 11 shown in Fig. 1(b) to a portion corresponding to the luer adapter 32 shown in Fig. 6 and integrating them. Therefore, by using in such a manner that a female screw 43d provided in a lower part of the hub 43 is screwed into the female screw 31c of the blood sampling holder 31, as shown in Fig. 8, backflow is securely prevented as is the case of the embodiment shown in Fig. 6.

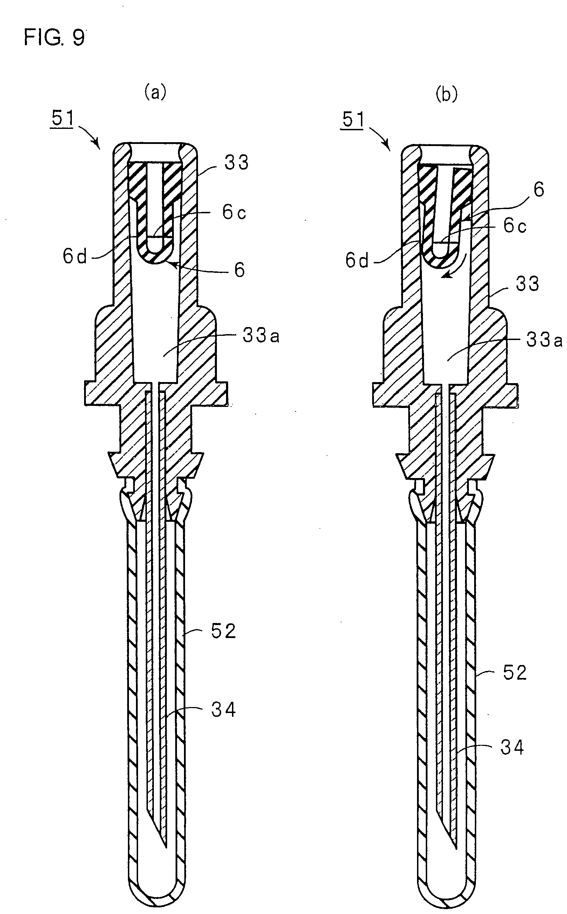

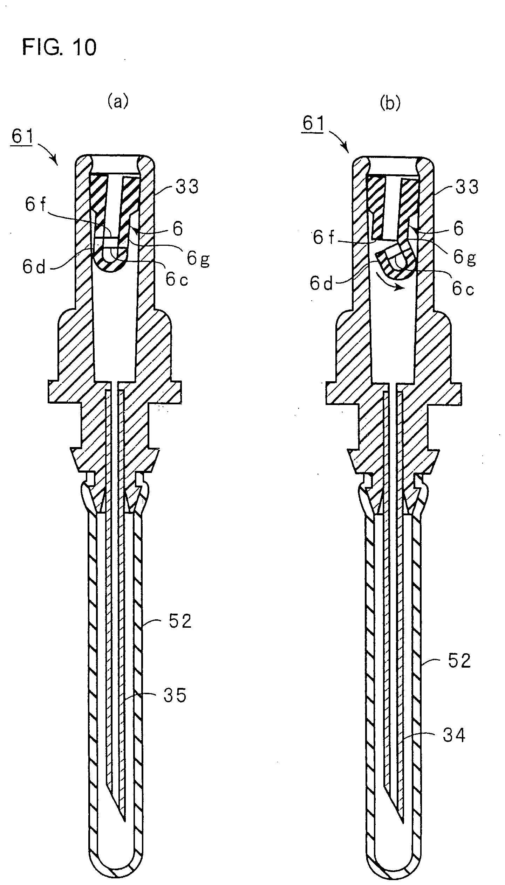

Figs. 9(a) and 9(b) are schematic front section views showing a luer adapter 51 according to a fourth embodiment of the present invention, and Figs. 10(a) and 10(b) are schematic front section views of a luer adapter according to a fifth embodiment which is more preferable than the fourth embodiment.

The luer adapter 51 shown in Figs. 9(a) and 9(b) is designed almost similarly to the luer adapter 32 shown in Fig. 6. Accordingly, detailed explanation for an equivalent part is omitted by denoting it by the same reference numeral and referring the explanation made with reference to Fig. 6.

A significant difference between the luer adapter 51 and the luer adapter 32 is that a rubber sheath 52 is provided so as to cover the hollow needle 34. The rubber sheath 52 is made of a material similar to that of the rubber sheath 5 shown in Fig. 1(a), and provided for a similar purpose.

Also in the luer adapter 51, the backflow preventing member 6 is pressed into the hub 33, and the backflow preventing member 6 is formed with the cut 6c. Therefore, the luer adapter 51 may be used similarly to the luer adapter 32 described above.

However, the backflow preventing member 6 may possibly be secured while being inclined relative to the extending direction of the internal channel as shown in Fig. 9(b) by mistake. In such a case, depending on the inclination angle, a part including a vertically connected hinge part 6d may be in abutment with an inner wall of the hub 33 so that it functions as a hinge in the cut 6c for opening/closing of the backflow preventing member. In this case, if the part below the cut 6c moves as shown by the arrow in Fig. 9(b) due to a pressure difference to act to make an opening, movement in the direction of the arrow is regulated because the part including the hinge part 6d is in abutment with the inner wall of the hub 33. Accordingly, there is a fear that an opening is not made even when a pressure difference is applied in the cut 6c.

In order to solve such a problem, it is preferable to secure the backflow preventing member 6 in a proper orientation within the hub 33 as shown in Fig. 9(a).

In a luer adapter 61 according to the fifth embodiment shown in Figs. 10(a) and 10(b), however, it is possible to allow blood to flow reliably even if the backflow preventing member 6 is secured aslant. That is, the luer adapter 61 is designed in the same manner as the luer adapter 51 except for the structure of the backflow preventing member 6. Therefore, detailed description of an equivalent will be omitted by denoting the same part by the same reference numeral.

In the backflow preventing member 6 of the luer adapter 61 shown in Figs. 10(a) and 10(b), a plurality of cuts 6c, 6f are formed at different height positions. The cut 6c and the cut 6f have hinge parts provided at different parts. In the present embodiment, a hinge part 6g of the cut 6f is provided at a position opposite to a hinge part 6d of the cut 6c with respect to the axial center of the backflow preventing member 6.

Therefore, when the backflow preventing member 6 is secured aslant, and the hinge part 6d of one of the cuts 6c is in abutment with the inner wall of the hub 33, as shown in Fig. 10(a), the hinge part 6g of the other cut is securely separated from the inner wall of the hub 33. Consequently, a pressure difference generates between an upper part and a lower part of the backflow preventing member 6. As a result, when blood is required to flow, the cut 6f is securely opened by a pressure difference to rapidly permit blood flow even when the cut 6c is not open.

Therefore, as is the case with the luer adapter 61 of the fourth embodiment, in the backflow preventing member of the present invention, the backflow preventing member 6 is preferably provided with the plural cuts 6c, 6f.

More preferably, when the plural cuts cut 6c, 6f are provided as in the above embodiment, a hinge part of at least one cut and a hinge part of at least other one cut are provided in circumferentially different positions of the backflow preventing member 6. However, even when the hinge parts are provided in the circumferentially same positions of the backflow preventing member 6, one of the hinge parts can be securely separated from the inner wall of the hub on the condition that the plural cuts are provided at different height positions.

Therefore, the hinge parts of the plural cuts are not necessarily provided in circumferentially different positions of the backflow preventing member 6.

In the fourth embodiment, two cuts 6c, 6f are provided, however, three or more cuts may be provided.

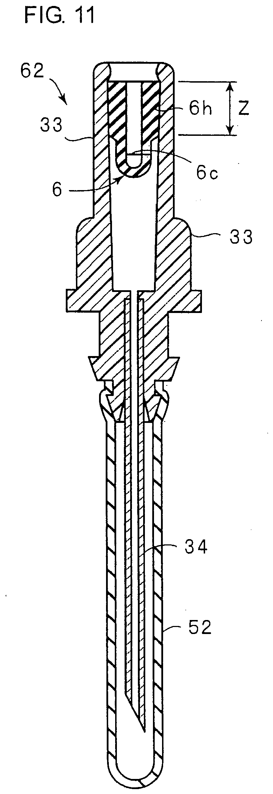

Fig. 11 is a front section view showing a luer adapter of the sixth embodiment of the present invention. A luer adapter 62 of the present embodiment has almost the same structure as the luer adapter 51. A difference is that in the luer adapter 62, a vertical dimension of a securing part 6h is made larger above the part where the cut 6c of the backflow preventing member 6 is provided. That is, in the luer adapter 62, a longitudinal dimension Z of the part where the outer circumferential surface of the backflow preventing member 6 is secured to the inner circumferential surface of the hub 33 is made sufficiently large. Consequently, in the luer adapter 62, when the backflow preventing member 6 is inserted into the hub 33, the backflow preventing member 6 is secured so that the orientation of the backflow preventing member 6 is correct. In other words, the backflow preventing member 6 is made unlikely to inline with respect to the extending direction of the internal channel.

Therefore, as is the case with the luer adapter 62 according to the sixth embodiment, it is preferred that a dimension along the extending direction of the internal channel, of the part where the outer circumferential surface of the backflow preventing member 6 is brought into close contact with the inner circumferential surface of the internal channel is set to at such a length that restricts the inclination as described above.

In the aforementioned embodiment, the blood sampling holder has a circular tubular shape, however, it may be other tubular shapes such as rectangular tubular shape.

The cut 6c formed in the backflow preventing member 6 should extends in the direction that intersects with the direction connecting the first and the second end parts, but not necessarily be provided in the direction perpendicular to the direction connecting the first and the second end parts as is the case of the above embodiment. Nevertheless, the cut 6c is preferably formed in the direction perpendicular to the direction connecting the first and the second end parts of the internal channel from the view point of securely closing the cut 6c by backflow.

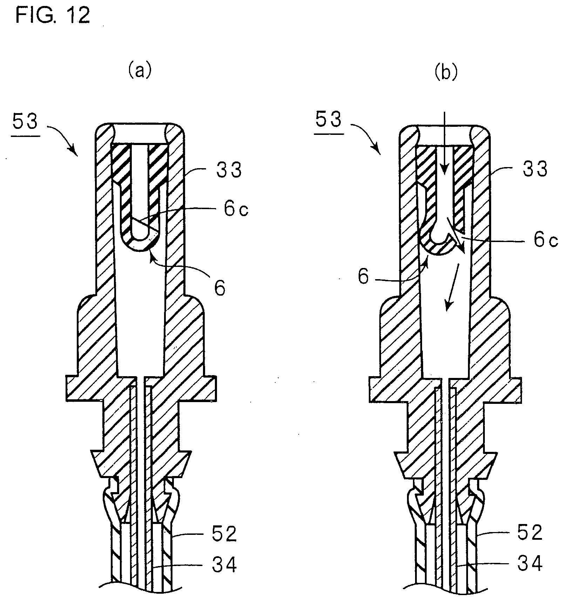

Figs. 12(a) and 12(b) are partially cutaway front section views showing an alternate example of the luer adapter 51 shown in Fig. 9(a). In a luer adapter 53 according to this alternate example, the direction in which the cut 6c provided in the backflow preventing member 6 extends is not perpendicular to the extending direction of the internal channel, or to the direction connecting the first and the second end parts, but extends in a direction inclined at about 60 degrees. Therefore, when the cut 6c is open due to a pressure difference, a sufficient area of the opening part is obtained as shown in Fig. 12(b). Therefore, when the cut 6c is opened due to a pressure difference and blood flows, it is possible to reduce resistance of blood flow to sufficiently small. Also it is possible to regulate extension of a blood sampling time caused by application of the backflow preventing structure.

In this alternate example, the extending direction of the cut 6c is inclined at an angle of 60 degrees with respect to the direction connecting the first and the second end parts, i.e. the direction in which the internal channel extends. Appropriate inclination at an angle ranging from 15 degrees to 165 degrees as well as at an angle of 60 degrees will realize the effect of increasing the opening area of the part where the cut 6c opens.

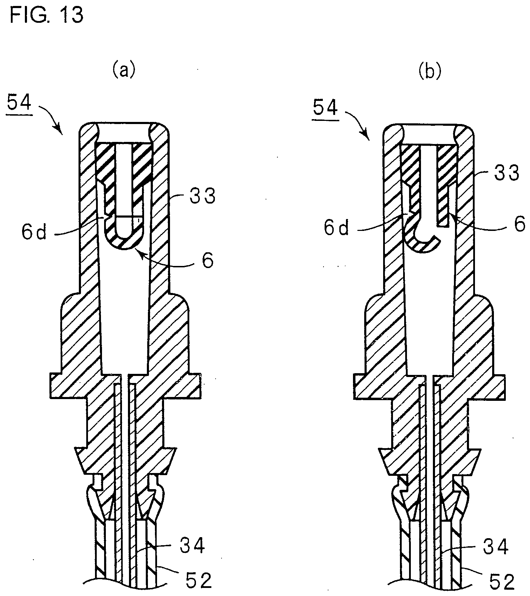

Figs. 13(a) and 13(b) are partial cutaway front section views showing a luer adapter according to a further alternate example of the luer adapter 51.

According to a luer adapter 54 shown in Fig. 13(a), in the backflow preventing member 6, the cut 6c is formed similarly to the case of the luer adapter 51. In the part where the cut 6c is provided, the thickness of the hinge part 6d when the cut 6c is opened due to a pressure difference as shown in Fig. 13(b) is made smaller. That is, the hinge part 6d corresponds to a part that supports an opening formed as described above when the cut 6c is open.

As shown in Fig. 13(a), when the cut 6c is provided in the direction perpendicular to the extending direction of the internal channel, the hinge 6d is provided at the same height position as the cut 6c. Since the thickness of the hinge part 6d is relatively smaller than that of the peripheral part, the cut 6c part is securely opened as shown in Fig. 13(b) when only a slight pressure difference is generated. Accordingly, it is possible to securely sample blood even when a blood sampling tube with poor absorbing ability is connected. Furthermore, since only the hinge part 6d should be made relatively thin, the contacting area of the part where the cut 6c is formed can be sufficiently ensured. That is, by making the thickness of the hinge part 6d relatively small and making the thickness of the part opposing via the cut 6c relatively large, the area of contacting part by the cut 6c can be sufficiently large. Therefore, even if the positions of the upper part and the lower part of the cut 6c circumferentially misalign when the cut 6c is closed by a backflow preventing operation after it is opened, it is possible to securely close the flow path.

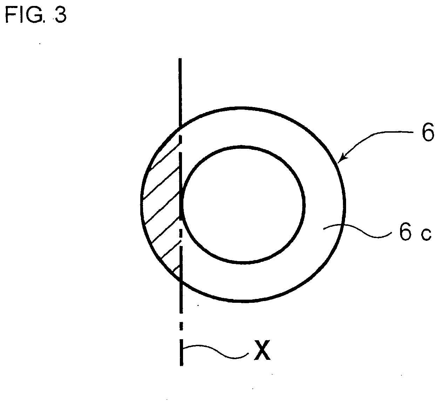

The amount of cut formed in the backflow preventing member 6, that is, the size of the cross section of the cut part formed by cutting may be adjusted depending on hardness and thickness of the backflow preventing member. A preferred example of such size of the cut 6c will be explained with reference to Fig. 3. Fig. 3 is a plan section view of a part where the cut 6c is formed in the backflow preventing member 6, wherein the cut 6c is formed by cutting. At the illustrated height position, the backflow preventing member is vertically successive in part other than the part of the cut 6c. The successive part 6d is marked with hatched lines in Fig. 3. Accordingly, in Fig. 3, the part that is not marked with hatched lines constitutes the cut 6c.

Taking as a standard the case where an elastic material formed of butyl rubber having a Shore A hardness ranging from 40 to 45, and a thickness of about 0.8 mm is used, it is preferred that the cut 6c has a cut part which is one-half or more of a traverse section of the backflow preventing member 6 as shown in Fig. 3. In the traverse section shown in Fig. 3, in particular, it is preferred that the cut 6c is formed by cutting to a tangential line X that contacts the inner circumferential surface. When the hardness of the material forming the backflow preventing member 6 is large, or when the thickness of the backflow preventing member 6 is large, the force of closing the opened part can be increased by reducing the size of the cut 6c, and the effect of preventing backflow is improved. However, there is a fear that a desired amount of blood cannot be sampled because of extended blood sampling time due to reduction in flow rate, or because the absorbing ability of blood based on a pressure difference is hard to act in vacuum blood sampling. To the contrary, when the hardness is small, thickness is small, or the cut 6c is too large, an opening is readily made, and the backflow preventing effect may be deteriorated although blood sampling time and blood sampling amount are hard to be influenced. Therefore, the hardness, thickness of the backflow preventing member 6, and the size of the cut 6c may be adjusted from these points of view.

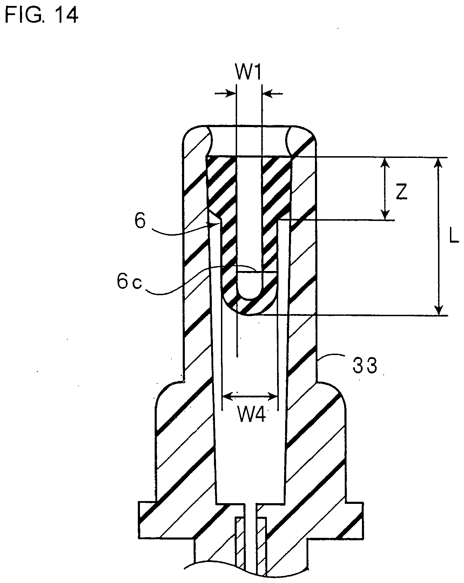

According to the "Standard for sterile injection syringe" announced by Ministry of Health, Labor and Welfare of Japan, in the luer adapter 51 as shown in Fig. 9, for example, blood sampling may be realized even with a blood sampling tube of weak aspiration ability insofar as an inner diameter W1 of the backflow preventing member 6 is equal to or more than 0.1 mm as shown in Fig. 14 in an enlarged scale. However, it is more preferable that the inner diameter W1 is equal to or more than 0.2 mm. When the inner diameter W1 is less than 0.2 mm, the resistance for blood flow tends to be large, which may increase the time required for bloods sampling. Therefore, in order to reduce the load of an operator such as physician or clinical technologist, the inner diameter W1 is preferably made large.

In general, the backflow preventing member 6 has a thickness ranging from 0.1 to 1.0 mm in the vicinity of the cut 6c. If the thickness is less than 0.1 mm, when it closes during a backflow preventing operation, the contacting surface in the cut 6c misaligns, which may hinder prevention of backflow. To the contrary, if the thickness is more than 1.0 mm, the force necessary to close for prevention of backflow is large, so that there is a fear that closing occurs before the sampling amount of blood reaches a predetermined amount. In practice, when an outer diameter of a luer adapter W2 is 4.0 mm, an inner diameter W3 is 3.0 mm, and when it is desired that a gap between the inner circumferential surface of the hub 33 and the backflow preventing member 6 is 0.5 mm, the outer diameter of the backflow preventing member 6 should be 2.0 mm. Therefore, it is practically difficult to provide a backflow preventing member 6 having a thickness larger than 1.0 mm according to the above dimensional relationship.

The facts that the inner diameter of the backflow preventing member 6 is preferably 0.2 mm or larger, and that the thickness is preferably in the range of 0.1 to 1.0 mm premise the luer adapter in conformance with the aforementioned "Standard for sterile injection syringe" announced by Ministry of Health, Labor and Welfare of Japan. When dimensions of a luer adapter and a blood sampler are different from those described above, the above dimensions also change accordingly. Next, concrete test examples producing luer adapter 51 of various dimensions will be explained.

### (Example 1)

A luer adapter having a dimension in conformance with the "Standard for sterile injection syringe" announced by Ministry of Health, Labor and Welfare of Japan was prepared. To be more specific, a backflow preventing member 6 having an overall length L of 6.0 mm, an outer diameter W4 of 2.0 mm, an inner diameter W1 of 1.0 mm, and a thickness of 0.5 mm was set with respect to a hub having an inner diameter of 2.5 in the hub 33 shown in Fig. 14. A holding part where the outer circumferential surface of the backflow preventing member 6 was in close contact with the inner circumferential surface of the hub 33 had a length Z of 2.0 mm, and an outer diameter of 2.9 mm, and a longitudinal dimension of the cut 6c was 1.5 mm. The backflow preventing member 6 was made of natural rubber, and the direction in which the cut 6c extends was perpendicular to the direction connecting the first and the second end parts.

### (Example 2)

Example 1 was followed except that the part where the cut 6c of the backflow preventing member was provided had an outer diameter W4 of 1.8 mm, an inner diameter W1 of 1.2 mm, and a thickness of 0.3 mm.

### (Example 3)

Example 1 was followed except that the part where the cut 6c of the backflow preventing member 6 was provided had an outer diameter W4 of 1.8 mm, an inner diameter W1 of 1.2 mm, and a thickness of 0.3 mm, and that the cut 6c was inclined at an angle of 45 degrees with respect to the direction connecting the first and the second end parts which is the extending direction of the internal channel.

### (Example 4)

Example 1 was followed except that the backflow preventing member 6 had an outer diameter W4 of 1.8 mm, an inner diameter W1 of 1.2 mm, and a thickness of 0.3 mm, and that two cuts were provided that extend horizontally in different height positions, i.e., extend in the direction perpendicular to the extending direction of the internal channel.

### (Comparative example)

A luer adapter having a hub as same as Example 1 except that the backflow preventing member was not inserted was prepared.

With respect to the luer adapter of Examples 1 to 4 and Comparative example, a backflow amount and an amount of absorbed water were evaluated in the following manner. The results are shown in Table 1.

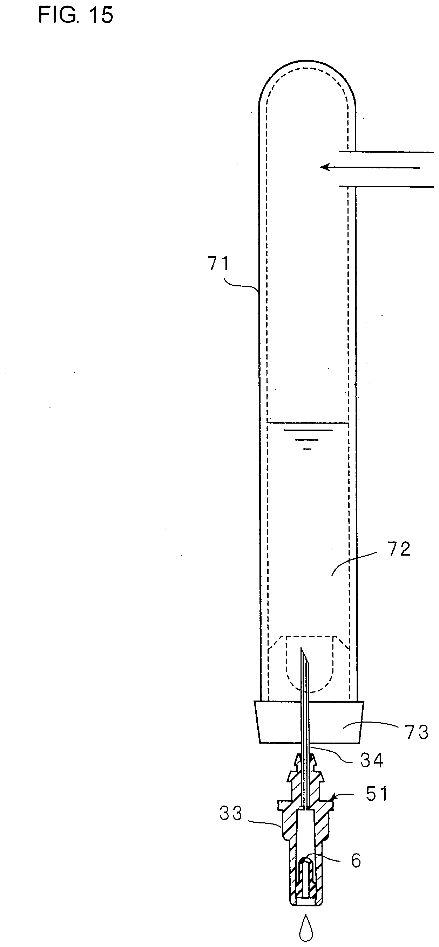

Backflow amount: As shown in Fig. 15, a blood sampling tube 71 was charged with water 72, and a pressure of 100 mmHg was added to the blood sampling tube 71. A plug member 73 of the blood sampling tube 71 was pierced with a hollow needle of the luer adapter 51 and left for 60 seconds. In this case, the blood sampling tube 71 was vertically inverted as illustrated, and an amount of water leaking out below the luer adapter 51 after leaving for 60 seconds was measured as a backflow amount.

Weight of collected water: A test tube with a capacity of 7 cc was sealed with a rubber plug under a reduced pressure, and a vacuum blood sampling tube was prepared. The above luer adapter was attached to the blood sampling holder. A burette was filled with water, and a tub of the burette was opened. The water in the burette through which a spring clip is passed was made to flow into a silicone tube connected to the burette, and the water flow was stopped by clipping at a burette scale of 0.

A hollow needle outside the blood sampling holder to which the luer adapter was attached was pierced into a silicone tube, and a needlepoint was placed within the silicone tube. Then on the side of the opposite hollow needle of the blood sampling holder, the aforementioned blood sampling tube was inserted, and water absorption was started by a pressure difference. After the interior of the blood sampling tube was filled with water, it was left for at least one minute. Thereafter, the highest water surface of the burette was made coincidence with the level of the liquid surface of the blood sampling tube at an accuracy of ±0.1 mL, and then an amount of absorption was determined.

**[Table 1]**

| | Backflow Amount (mg) | Amount of Water Absorption (g) |
|---|---|---|
| Example 1 | 0.0 | 1.92±0.075 |
| Example 2 | 0.0 | 1.95±0.031 |
| Example 3 | 0.0 | 1.90±0.061 |
| Example 4 | 0.0 | 1.95±0.042 |
| Comparative Example | --- | 2.00±0.020 |

As is apparent from Table 1, Examples 1 to 4 securely prevent backflow in comparison with Comparative example. Also the amount of water absorption falls within the range of 95 to 97.5% by weight based on 100% for Comparative example, and reduction in amount of water absorption resulting from insertion of the backflow preventing member 6 was not observed.

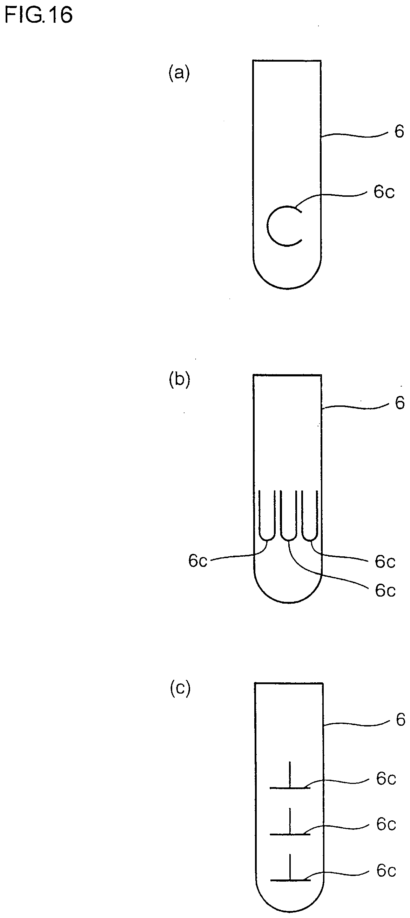

In the above Examples, a linear cut is formed in the backflow preventing member 6, however, the shape and number of cut provided in the backflow preventing member according to the present invention may be appropriately changed. Such alternate examples are exemplarily shown in Figs. 16(a) to 16(c). More specifically, in the backflow preventing member 6 shown in Figs. 16(a) to 16(c), at least one cut 6c is formed in various shapes such as curve, or a series of lines that extend in different directions. As described above, it is to be noted that in the present invention, the shape and number of cut formed in the backflow preventing member is not particularly limited.

## Claims

1. A backflow preventing structure of a blood sampler, for preventing backflow of blood in the blood sampler, the structure comprising:
- a tubular body having an internal channel (3a) extending from a first end part (3b) toward a second end part (3c) through which blood flows; and
- a backflow preventing member (6) contained in the internal channel (3a) of the tubular body, made of a material having rubber elasticity, the backflow preventing member (6) being a bottomed tube convexly semispherically closed at the second end part side (3c) and having, subsequent to the convex semispherical bottom end, a cylindrical main body part (6b) and a thickened part (6a) subsequent to the main body part (6b) with an opening that opens toward the first end part (3b), wherein the thickened part (6a) has an extended outer diameter when compared to the main body part (6b);
wherein the extended outer diameter of the thickened part (6a) is selected so that an outer circumferential surface of the thickened part (6a) comes into close liquid-tight contact with an inner circumferential surface of the internal channel (3a);
- a cut (6c) in the cylindrical main body part (6b) of the backflow preventing member (6) at a part closer to the second end part (3c) side than the thickened part, wherein the cut (6c) extends in a direction that intersects with a direction connecting the first end part (3b) and the second end part (3c), and
- a hinge part (6d) is provided within the cylindrical main body part (6b) of the backflow preventing member (6) for opening/closing achieved by the cut (6c), wherein the thickness of the hinge part (6d) is smaller than that of the peripheral part of the cylindrical main body part (6b) by a notch provided in the outer wall of the cylindrical main body part (6b), wherein the hinge part (6d) is provided at a part of the cylindrical main body part (6b) opposed to and at the same height position as the cut (6c).

2. The backflow preventing structure according to claim 1, wherein the cut (6c) is provided in at least two positions.

3. The backflow preventing structure according to claim 1, wherein the cut (6c) formed in the backflow preventing member (6) extends in a direction inclined at an angle ranging from 15 to 165 degrees with respect to the direction connecting the first end part (3b) and the second end part (3c).

4. The backflow preventing structure according to claim 1, wherein the cut (6c) formed in the backflow preventing member (6) extends in a direction perpendicular to the direction connecting the first end part (3b) and the second end part (3c).

5. A luer adapter (32) comprising:
- a hollow needle (34) used for piercing a plug member of a blood sampling tube (31) in blood sampling, and
- a hub (33) secured to one end of the hollow needle (34),
wherein the hub (33) has the backflow preventing structure according to any one of claims 1 to 4, and the hollow needle (34) is in communication with the second end part side (33c) of the internal channel (33a) of the backflow preventing structure of a blood sampler.

6. A blood sampling needle (41) comprising:
- a hollow needle (44) for collecting blood; and
- a hub (43) secured to one end of the hollow needle (44),
wherein the hub (43) has the backflow preventing structure according to any one of claims 1 to 4, and the hollow needle (44) is in communication with the first end part side (43b) of the internal channel (43a) of the backflow preventing structure of a blood sampler.

7. A blood sampling holder (1; 31) comprising:
- a tubular holder main body (2) formed with an opening (2a; 31a) for inserting a blood sampling tube at its one end, and closed (2b; 31b) at the other end;
- a hub (3; 43) provided on the other end side of the holder main body (2); and
- a hollow needle (4; 41) secured at its one end (45) to the hub (3; 43), for piercing a plug member of the blood sampling tube,
wherein the hub (3; 43) has the backflow preventing structure according to any one of claims 1 to 4, and the hollow needle (4; 45) is in communication with the second end part side (3c; 43c) of the internal channel (3a; 43a) of the backflow preventing structure of a blood sampler.

8. The blood sampling holder (31) according to claim 7, further comprising
- a blood sampling hollow needle (41) having one end (45) secured to the hub (43) and the other end (44) extended outside the holder (31),
wherein the blood sampling hollow needle (41) is in communication with the first end part side (43b) of the internal channel (43a) of the backflow preventing structure.

## Patentansprüche

1. Rückflussverhinderungsstruktur für einen Blutprobennehmer zum Verhindern eines Rückflusses von Blut in dem Blutprobennehmer, wobei die Struktur Folgendes umfasst:
- einen rohrförmigen Körper, der einen inneren Kanal (3a) aufweist, der sich von einem ersten Endteil (3b) zu einem zweiten Endteil (3c) erstreckt, durch den Blut strömt; und
- ein Rückflussverhinderungselement (6), das in dem inneren Kanal (3a) des rohrförmigen Körpers enthalten ist, das aus einem Material hergestellt ist, das eine Gummielastizität aufweist, wobei das Rückflussverhinderungselement (6) ein mit einem Boden versehenes Rohr ist, das auf der Seite (3c) des zweiten Endteils konvex halbkugelförmig geschlossen ist und das nach dem konvexen halbkugelförmigen unteren Ende ein zylinderförmiges Hauptkörperteil (6b) und ein verdicktes Teil (6a) nach dem Hauptkörperteil (6b) mit einer Öffnung aufweist, die sich in Richtung des ersten Endteils (3b) öffnet, wobei das verdickte Teil (6a) einen erweiterten Außendurchmesser im Vergleich zu dem Hauptkörperteil (6b) aufweist;
wobei der erweiterte Außendurchmesser des verdickten Teils (6a) so gewählt ist, dass eine Außenumfangsfläche des verdickten Teils (6a) in direkten flüssigkeitsdichten Kontakt mit einer Innenumfangsfläche des inneren Kanals (3a) gelangt;
- einen Einschnitt (6c) in dem zylinderförmigen Hauptkörperteil (6b) des Rückflussverhinderungselements (6) bei einem Teil, das näher an der Seite des zweiten Endteils (3c) als bei dem verdickten Teil liegt, wobei sich der Einschnitt (6c) in einer Richtung erstreckt, die mit eine Richtung schneidet, die das erste Endteil (3b) und das zweite Endteil (3c) verbindet, und
- ein Gelenkteil (6d), das in dem zylinderförmigen Hauptkörperteil (6b) des Rückflussverhinderungselements (6) zum Öffnen bzw. Schließen vorgesehen ist, das durch den Einschnitt (6c) realisiert wird, wobei die Dicke des Gelenkteils (6d) kleiner als die des Umfangsteils des zylinderförmigen Hauptkörperteils (6b) ist, durch eine Kerbe, die in der Außenwand des zylinderförmigen Hauptkörperteils (6b) vorgesehen ist, wobei das Gelenkteil (6d) an einem Teil des zylinderförmigen Hauptkörperteils (6b) gegenüber dem Einschnitt (6c) und auf der gleichen Höhenposition vorgesehen ist.

2. Rückflussverhinderungsstruktur nach Anspruch 1, wobei der Einschnitt (6c) bei wenigstens zwei Positionen vorgesehen ist.

3. Rückflussverhinderungsstruktur nach Anspruch 1, wobei sich der Einschnitt (6c), der in dem Rückflussverhinderungselement (6) ausgebildet ist, in einer Richtung erstreckt, die unter einem Winkel geneigt ist, der im Bereich von 15 bis 165 Grad in Bezug auf die Richtung liegt, die das erste Endteil (3b) und das zweite Endteil (3c) verbindet.

4. Rückflussverhinderungsstruktur nach Anspruch 1, wobei sich der Einschnitt (6c), der in dem Rückflussverhinderungselement (6) ausgebildet ist, in einer Richtung erstreckt, die senkrecht zu der Richtung ist, die das erste Endteil (3b) und das zweite Endteil (3c) verbindet.

5. Luer-Adapter (32), der Folgendes umfasst:
- eine Hohlnadel (34), die zum Durchstechen eines Pfropfenelements eines Blutprobenentnahmerohrs (31) bei einer Blutentnahme verwendet wird, und
- eine Nabe (33), die an einem Ende der Hohlnadel (34) befestigt ist,
wobei die Nabe (33) die Rückflussverhinderungsstruktur nach einem der Ansprüche 1 bis 4 aufweist und wobei die Hohlnadel (34) mit der Seite (33c) des zweiten Endteils des inneren Kanals (33a) der Rückflussverhinderungsstruktur eines Blutprobennehmers in Kommunikation ist.

6. Blutentnahmenadel (41), die Folgendes umfasst:
- eine Hohlnadel (44) zum Sammeln von Blut; und
- eine Nabe (43), die an einem Ende der Hohlnadel (44) befestigt ist,
wobei die Nabe (43) die Rückflussverhinderungsstruktur nach einem der Ansprüche 1 bis 4 aufweist und wobei die Hohlnadel (44) mit der Seite (43b) des ersten Endteils des inneren Kanals (43a) der Rückflussverhinderungsstruktur eines Blutprobennehmers in Kommunikation ist.

7. Blutentnahmehalterung (1; 31), die Folgendes umfasst:
- einen rohrförmigen Halterungshauptkörper (2), der mit einer Öffnung (2a; 31a) zum Einsetzen eines Blutentnahmerohrs an einem Ende ausgebildet ist und an dem anderen Ende geschlossen ist (2b; 31b);
- eine Nabe (3; 43), die an der Seite des anderen Endes des Halterungshauptkörpers (2) vorgesehen ist; und
- eine Hohlnadel (4; 41), die an ihrem einen Ende (45) an der Nabe (3; 43) befestigt ist, zum Durchstechen eines Pfropfenelements des Blutentnahmerohrs,
wobei die Nabe (3; 43) die Rückflussverhinderungsstruktur nach einem der Ansprüche 1 bis 4 aufweist und wobei die Hohlnadel (4; 45) mit der Seite (3c; 43c) des zweiten Endteils des inneren Kanals (3a; 43a) der Rückflussverhinderungsstruktur eines Blutprobennehmers in Kommunikation ist.

8. Blutentnahmehalterung (31) nach Anspruch 7, die ferner Folgendes umfasst:
- eine Blutentnahme-Hohlnadel (41), wovon ein Ende (45) an der Nabe (43) befestigt ist und sich das andere Ende (44) zur Außenseite der Halterung (31) erstreckt,
wobei die Blutentnahme-Hohlnadel (41) mit der Seite (43b) des ersten Endteils des inneren Kanals (43a) der Rückflussverhinderungsstruktur in Kommunikation ist.

## Revendications

1. Structure antireflux d'un dispositif de prélèvement sanguin, pour empêcher un reflux de sang dans le dispositif de prélèvement sanguin, la structure comprenant :
- un corps tubulaire comportant un canal interne (3a) s'étendant d'une première partie d'extrémité (3b) vers une deuxième partie d'extrémité (3c) à travers lequel le sang s'écoule ; et
- un organe antireflux (6) contenu dans le canal interne (3a) du corps tubulaire, constitué d'un matériau présentant une élasticité de caoutchouc, l'organe antireflux (6) étant un tube comportant un fond fermé de manière hémisphérique convexe au côté de deuxième partie d'extrémité (3c) et comportant, à la suite de l'extrémité de fond hémisphérique convexe, une partie de corps principal cylindrique (6b) et une partie épaissie (6a) à la suite de la partie de corps principal (6b) avec une ouverture qui s'ouvre vers la première partie d'extrémité (3b), dans laquelle la partie épaissie (6a) présente un diamètre extérieur étendu par comparaison avec la partie de corps principal (6b) ;
dans laquelle le diamètre extérieur étendu de la partie épaissie (6a) est sélectionné de sorte qu'une surface circonférentielle extérieure de la partie épaissie (6a) vienne en contact étroit étanche aux liquides avec une surface circonférentielle intérieure du canal interne (3a) ;
- une coupure (6c) dans la partie de corps principal cylindrique (6b) de l'organe antireflux (6) à une partie plus proche du côté de deuxième partie d'extrémité (3c) que de la partie épaissie, dans laquelle la coupure (6c) s'étend dans un sens en intersection avec un sens reliant la première partie d'extrémité (3b) et la deuxième partie d'extrémité (3c), et
- une partie de charnière (6d) est prévue à l'intérieur de la partie de corps principal cylindrique (6b) de l'organe antireflux (6) pour une ouverture/fermeture réalisée par la coupure (6c), dans laquelle l'épaisseur de la partie de charnière (6d) est inférieure à celle de la partie périphérique de la partie de corps principal cylindrique (6b) par une encoche prévue dans la paroi extérieure de la partie de corps principal cylindrique (6b), dans laquelle la partie de charnière (6b) est prévue à une partie de la partie de corps principal cylindrique (6b) à l'opposé de la coupure (6c) et à la même position de hauteur que la coupure (6c).

2. Structure antireflux selon la revendication 1, dans laquelle la coupure (6c) est prévue à au moins deux positions.

3. Structure antireflux selon la revendication 1, dans laquelle la coupure (6c) formée dans l'organe antireflux (6) s'étend dans un sens incliné à un angle dans une plage de 15 à 165 degrés par rapport au sens reliant la première partie d'extrémité (3b) et la deuxième partie d'extrémité (3c).

4. Structure antireflux selon la revendication 1, dans laquelle la coupure (6c) formée dans l'organe antireflux (6) s'étend dans un sens perpendiculaire au sens reliant la première partie d'extrémité (3b) et la deuxième partie d'extrémité (3c).

5. Adaptateur Luer (32) comprenant :
- une aiguille creuse (34) utilisée pour percer un organe de bouchon d'un tube de prélèvement sanguin (31) au cours d'un prélèvement sanguin, et
- une embase (33) fixée à une extrémité de l'aiguille creuse (34),
dans laquelle l'embase (33) comporte la structure antireflux selon l'une quelconque des revendications 1 à 4, et l'aiguille creuse (34) est en communication avec le côté de deuxième partie d'extrémité (33c) du canal interne (33a) de la structure antireflux d'un dispositif de prélèvement sanguin.

6. Aiguille de prélèvement sanguin (41) comprenant :
- une aiguille creuse (44) pour recueillir du sang ; et
- une embase (43) fixée à une extrémité de l'aiguille creuse (44),
dans laquelle l'embase (43) comporte la structure antireflux selon l'une quelconque des revendications 1 à 4, et l'aiguille creuse (44) est en communication avec le côté de première partie d'extrémité (43b) du canal interne (43a) de la structure antireflux d'un dispositif de prélèvement sanguin.

7. Porte-prélèvement sanguin (1 ; 31) comprenant :
- un corps principal de porte-prélèvement tubulaire (2) formé avec une ouverture (2a ; 31a) pour introduire un tube de prélèvement sanguin à une extrémité, et fermé (2b ; 31b) à l'autre extrémité ;
- une embase (3 ; 43) prévue sur le côté d'autre extrémité du corps principal de porte-prélèvement (2) ; et
- une aiguille creuse (4 ; 41) fixée à une extrémité (45) à l'embase (3 ; 43), pour percer un organe de bouchon du tube de prélèvement sanguin,
dans lequel l'embase (3 ; 43) comporte la structure antireflux selon l'une quelconque des revendications 1 à 4, et l'aiguille creuse (4 ; 45) est en communication avec le côté de deuxième partie d'extrémité (3c ; 43c) du canal interne (3a ; 43a) de la structure antireflux d'un dispositif de prélèvement sanguin.

8. Porte-prélèvement sanguin (31) selon la revendication 7, comprenant en outre :
- une aiguille creuse de prélèvement sanguin (41) comportant une extrémité (45) fixée à l'embase (43) et l'autre extrémité (44) étendue à l'extérieur du porte-prélèvement (31),
dans lequel l'aiguille creuse de prélèvement sanguin (41) est en communication avec le côté de première partie d'extrémité (43b) du canal interne (43a) de la structure antireflux.
